# EUROPÄISCHE PATENTSCHRIFT

(11) **EP 4 149 573 B1**
(45) Veröffentlichungstag und Bekanntmachung des Hinweises auf die Patenterteilung: **28.01.2026**
(21) Anmeldenummer: 21727784.7
(22) Anmeldetag: 12.05.2021
(51) Int. Cl.: A61L 9/20, A62B 18/08, A62B 23/02

(54) **ATEMLUFTDESINFEKTIONSVORRICHTUNG, ATEMSCHUTZMASKE DAMIT UND ATEMLUFTDESINFEKTIONSVERFAHREN DAMIT**
DEVICE FOR DISINFECTING BREATHING AIR, MASK WITH SUCH A DEVICE, AND METHOD FOR DISINFECTING BREATHING AIR WITH SUCH A DEVICE
DISPOSITIF DE DÉSINFECTION D'AIR RESPIRATOIRE, MASQUE DE PROTECTION RESPIRATOIRE ÉQUIPÉ DE CE DERNIER ET PROCÉDÉ DE DÉSINFECTION D'AIR RESPIRATOIRE L'UTILISANT

(30) Priorität: 13.05.2020 DE 102020112999
(43) Veröffentlichungstag der Anmeldung: 22.03.2023
(73) Patentinhaber: Höhne, Bernd, 28832 Achim (DE)
(72) Erfinder: Höhne, Bernd, 28832 Achim (DE)
(74) Vertreter: Hansen, Jochen
(86) Internationale Anmeldenummer: PCT/EP2021/062703
(87) Internationale Veröffentlichungsnummer: WO 2021/228984

(56) Entgegenhaltungen:
- US-A1- 2015 359 921
- US-A1- 2016 001 108
- US-A1- 2019 117 820

## Beschreibung

Die Erfindung betrifft eine Atemluftdesinfektionsvorrichtung mit einem Atemluftkanal, über den Atemluft über einen Einlassabschnitt in einer ersten Fließrichtung einer Person zu- und/oder abgeführt wird, wobei ein verbreiterter Abschnitt des Atemluftkanals vorgesehen ist und im verbreiterten Abschnitt des Atemluftkanals UVC-Strahlungsmittel zur Desinfektion der Atemluft angeordnet sind. Ferner betrifft die Erfindung eine Atemschutzmaske mit derartigen Atemluftdesinfektionsvorrichtungen und ein Atemluftdesinfektionsverfahren damit.

Dabei ist die erste Fließrichtung der Atemluft als eine generelle Fließrichtung zu verstehen, die entlang der Längsachse des Atemluftkanals, der beispielsweise als Atemluftschlauch oder Dergleichen ausgebildet ist, zu einer Person hin- oder von dieser wegführt.

Aus der US 2016/0001108 A1 ist eine Atemluftdesinfektionsvorrichtung bekannt, die in einer Strömungskammer UV-Bestrahlungsmittel aufweist, die die durchströmende Luft desinfizieren. Dabei wird die Atemluft über eine Strömungskammer geführt und dort durch Strukturen verwirbelt, sodass die Luftströmungsbahn effektiv verlängert wird. Alternativ ist die Strömungskammer in mehrere serpentinenartige Atemluftkanäle unterteilt, die den Luftweg verlängern und somit die Luftreinigung verbessern.

Die nachveröffentlichte DE 10 2020 106 235 B3 beschreibt eine Atemschutzmaske, bei der Atemluft durch einen Kammerinnenraum geführt wird, in dem ultraviolett emittierende Lichtquellen angeordnet sind. Der Kammerinnenraum kann in zwei Kammerabschnitte mittels Trenneinrichtung ausgebildet sein, wobei die Kammerabschnitte fluidisch verbunden sind.

Aus der US 2019/0117820 A1 ist eine persönliche Luftbehandlungsvorrichtung bekannt, die insbesondere zusammen mit einer Partikelmaske zu verwenden ist, wobei die Luftbehandlungsvorrichtung eine LED UV-Lichtquelle zur Luftbehandlung ausweist.

Ferner ist in der US 2015/0359921 A1 ein Luftreinigungssystem für Gebäude beschrieben, bei dem über einen Ventilator angesogene Außenluft durch eine Bestrahlungskammer mit UV-Lichtquelle mit keimtötender Wirkung geleitet wird. Dabei ist in einer besonderen Ausführungsform ein Ablenkblech in Form einer flachen Platte ausgebildet, an deren Rändern Lücken vorgesehen sind, durch die die zuströmende Luft geleitet wird. Hinter dieser flachen Platte ist eine perforierte Platte angeordnet, um die in der UV-Kammer einwirkende UV-Strahlung abschirmen zu können.

Ausgehend von der US 2015/0359921 A1 ist es Aufgabe der Erfindung eine Atemluftdesinfektionsvorrichtung bzw. -verfahren anzugeben, mit der bzw. dem eine Desinfektion der Atemluft zuverlässig und energieeffizient möglich ist.

Gelöst wird diese Aufgabe mit einer Atemluftdesinfektionsvorrichtung gemäß Anspruch 1 und einem Atemluftdesinfektionsverfahren gemäß Anspruch 8.

Dadurch, dass der verbreiterte Abschnitt des Atemluftkanals eine allseitige Aufweitung in alle Radialrichtungen aufweist, wobei im Bereich der größten Aufweitung eine den geradlinigen Durchfluß der Atemluft hindernde Strömungsfläche vorgesehen ist, die eine Umlenkung der Atemluft in alle Radialrichtungen zur ersten Fließrichtung nach außen in diesem verbreiterten Abschnitt des Atemluftkanals bewirkt, und der somit wirksame Strömungsquerschnitt im verbreiterten Abschnitt gegenüber dem Strömungsquerschnitt im Einlassabschnitt aufgeweitet ist, wird der Strömungsweg der Atemluft in der Vorrichtung verlängert und gleichzeitig der Strömungsquerschnitt in diesem verbreiterten Abschnitt des Atemluftkanals vergrößert.

Dabei ist entscheidend, dass die Atemluft, die in der ursprünglichen ersten Fließrichtung (generelle Fließrichtung) fließt, nunmehr in alle Radialrichtungen umgelenkt wird, also nunmehr aufgefächert über die gesamte Umfangsrichtung um die erste Fließrichtung herum, in eine weitere, nämliche abgelenkte Fließrichtung als Radialfließrichtung umgelenkt wird, womit sich der wirksame Strömungsquerschnitt auf den gesamten Umfangmal der Breite dieses Bereiches aufteilt und in radialen Richtung nach außen mit dem zunehmenden Radius deutlich vergrößert.

Entsprechend wird die über diesen verbreiterten Abschnitt des Atemluftkanals geführte Atemluft in ihrer Strömungsgeschwindigkeit verlangsamt und der zurückzulegende Strömungsweg in diesem verbreiterten Abschnitt verlängert. Resultierend verlängert sich die Einwirkzeit und verbessert sich der Effekt der UVC-Strahlung auf die in diesem verbreiterten Abschnitt entlangströmende Atemluft und die darin möglicherweise enthaltenen Keime erheblich. Dabei ist zu berücksichtigen, dass UVC-Licht bereits in der Luft in seiner Intensität stark geschwächt wird, also nur eine Eindringtiefe von wenigen Millimetern bis 10 - 20 mm hat. Ferner ist zu berücksichtigen das etwaige dazwischenliegende Trennschichten eine zusätzliche, deutliche Abschwächung der Strahlungsintensität bewirken.

Ferner ist zu berücksichtigen, dass eine durchschnittliche erwachsene Person im Ruhestatus etwa einen halben Liter Luftvolumen als Atemluft binnen eines Atemzuges über eine Zeitdauer von ca. 1,5 s einatmet. Daraus ergibt sich für einen üblichen Atemluftzufuhrschlauch, wie er beispielsweise auch in Krankenhäusern verwendet wird, mit einem Innendurchmesser von 15 mm eine Strömungsgeschwindigkeit von ca. 2 m/s. Durch die Aufweitung des Strömungsquerschnittes im verbreiterten Abschnitt des Atemluftkanals wird im äußeren Peripheriebereich eine erheblich reduzierte Geschwindigkeit je nach geometrischer Ausgestaltung von wenigen cm/s bevorzugt 1 cm/s oder gar weniger, erreicht. Dafür ist der wirksame Strömungsquerschnitt im verbreiterten Abschnitt gegenüber dem Strömungsquerschnitt im Einlassabschnitt um den Faktor 10 bis 500, bevorzugt um den Faktor 20 bis 100, vergrößert.

Dabei hat die allseitige Aufweitung die Form eines Zylinders, wobei die Zylinderachse des Zylinders mit der ersten Fließrichtung zusammenfällt und die Strömungsfläche eine Kreisscheibe ist, die mittig und orthogonal zur ersten Fließrichtung im Zylinder so angeordnet ist, dass ein Ringraum zwischen Zylindermantelfläche und Kreisscheibe zur weiteren Strömungsumlenkung frei bleibt. Dabei erfolgt diese weitere Strömungsumlenkung im Bereich des Ringraums nahe dem äußeren Rand der Kreisscheibe, so dass hier die Atemluft - in noch verringerter Strömungsgeschwindigkeit - bereits in Richtung der ersten Fließrichtung abfließt. Je nach Ausbildung der Atemluftdesinfektionsvorrichtung und Weiterführung der Atemluft kann die Atemluft in einer Ausführungsform wieder in einem engeren einzelnen Atemluftschlauch als fortgeführter Atemluftkanal zusammengeführt werden, womit die Strömungsgeschwindigkeit der Atemluft wieder zunimmt, oder die Atemluft wird an dieser Stelle des Ringraumes zwischen Zylindermantelfläche und Kreisscheibe direkt oder radial geringfügig nach innen umgelenkt, beispielsweise in eine Atemmaske, geleitet.

Die Ausbildung der allseitigen Aufweitung in Form eines Zylinders ist fertigungstechnisch sehr einfach realisierbar und erlaubt eine strömungstechnisch weitgehend gleichmäßige Aufteilung der Atemluftströmung in diesem verbreiterten Abschnitt des Atemluftkanals. Entsprechend dürfte sich eine im Wesentlichen gleichmäßige Verringerung der Strömungsgeschwindigkeit und Verteilung der Atemluft in diesem Zylinder ergeben. Entsprechend dem dabei ebenfalls realisierten, verlängerten Strömungsweg, kann somit eine deutlich erhöhte Einwirkzeit der UVC-Bestrahlung auf die vorbeiströmende Atemluft erzielt werden.

Die UVC-Strahlungsmittel sind im verbreiterten Abschnitt im Bereich der größten Strömungsquerschnittsaufweitung angeordnet. Damit wirkt die UVC-Bestrahlung im Bereich der langsamsten Luftströmung, womit eine besonders hohe Wirksamkeit einhergeht. Berücksichtigt man nun den verlängerten Strömungsweg, die verlangsamte Luftströmung und die Möglichkeit an der Peripherie dieses verbreiterten Abschnitts des Atemluftkanals direkt die UVC-Strahlungsmittel anzuordnen, können effiziente Einwirkzeiten von 2 - 3 s der UVC-Bestrahlung auf ein in der Strömung vorbeifließendes Luftteilchen erreicht werden. Dabei konnte festgestellt werden, dass Einwirkzeiten von oberhalb 1 s bei der Verwendung von herkömmlichen UVC-LEDs mit beispielsweise 0,6 Watt Leistung eine sehr wirksame Keimreduzierung, nämlich Abtötung von etwaigen in der Luft transportierten Bakterien, Viren oder Dergleichen bewirkt.

Verfahrensgemäß wird diese Aufgabe mit den folgenden Schritten gelöst: Umlenken der Atemluft in alle Radialrichtungen zur ersten Fließrichtung nach außen in einem verbreiterten Abschnitt des Atemluftkanals, womit der Strömungsweg und der Strömungsquerschnitt in diesem verbreiterten Abschnitt des Atemluftkanals vergrößert wird, und Bestrahlen der Atemluft in diesem verbreiterten Abschnitt des Atemluftkanals mit UVC-Strahlung, wobei die Atemluft am radial-äußeren Rand im Bereich der größten Aufweitung des Strömungsquerschnittes der UVC-Strahlung ausgesetzt wird. Damit wird in diesem Bereich geringster Strömungsgeschwindigkeit der Luft sehr wirksam desinfiziert.

Wenn die Atemluft am radial-äußeren Rand in diesem verbreiterten Abschnitt des Atemluftkanals wieder in die generelle Fließrichtung rückumgelenkt wird, kann die Atemluft weiter über entsprechend angeschlossene Schläuche, wiederrum mit der darin sich ausbildenden deutlich höheren Luftströmungsgeschwindigkeit, zu einer Atemmaske geführt werden.

Alternativ können auch beispielsweise zwei Atemluftdesinfektionsvorrichtungen in einer Atemschutzmaske direkt angeordnet sein, wobei dann auf eine Rückumlenkung zurück auf die generelle Fließrichtung der Atemluft verzichtet und die so desinfizierte Atemluft direkt ins Innere der Atemmaske geleitet werden kann.

Ferner kann die UVC-Bestrahlung moduliert und/oder aufsummiert werden, um eine bessere Eindringtiefe der UVC-Bestrahlung in der zu desinfizierenden Atemluft zu erreichen. Damit soll der Dämpfung der UVC-Bestrahlung in der Atemluft entgegengewirkt werden, um eine verstärkende Wirkung der UVC-Bestrahlung zu gewährleisten. Hierbei kann eine bereits aus dem Funkbetrieb oder auch in der Modulation von Infrarot-Licht oder Laserlicht bekannte Technik angewendet werden. Dabei ist sowohl eine Amplituden- wie auch eine Frequenzmodulation möglich. Ferner können durch Hinzufügen von einer oder mehreren weiteren Lichtfrequenzen Summensignale mit UVC-verstärkender Wirkung erzeugt werden.

Zur Unterstützung einer gleichmäßigen Strömungsaufteilung sind Strömungsleitflächen im Atemluftkanal und insbesondere dem verbreiterten Abschnitt des Atemluftkanals vorgesehen, die die Strömung der Atemluft auf gleichartige Strömungswege aufteilt.

Wenn der verbreiterte Abschnitt des Atemluftkanals aus einem UVC-Licht durchlässigen Material, beispielsweise Kristallglas oder Graphene, insbesondere Kunststoff, bevorzugt PMME, gebildet ist, an dem als UVC-Strahlungsmittel UVC-LEDs angeordnet sind, können die UVC-LEDs auch außen an dem Gehäuse angeordnet werden. Alternativ können jedoch auch die UVC-Strahlungsmittel in Form von UVC-LEDs innerhalb des Atemluftkanals im verbreiterten Abschnitt angeordnet sein, da in diesem Fall eine direkte Bestrahlung der vorbeiströmenden Atemluft, ohne die Intensität der UVC-Bestrahlung durch Intensitäts-schwächende Grenzflächenübergänge berücksichtigen zu müssen, möglich ist.

Dadurch, dass ein Flüssigkeitsabscheider und/oder ein Partikelfilter im Atemluftkanal in Strömungsrichtung der Atemluft vor dem verbreiterten Abschnitt angeordnet ist, können unerwünschte Bestandteile, nämlich einerseits übermäßige Feuchtigkeit und andererseits etwaige Schmutz-/Staubpartikel aus der Atemluft gefiltert werden. Dies ist aus hygienischen Gründen vorteilhaft, da an Feuchtigkeitströpfchen und/oder Partikeln häufig Bakterien/Viren anlagern und eine Verschmutzung des Inneren der Atemluftdesinfektionsvorrichtung zu vermeiden ist.

Wenn ein Ultraschallgeber am verbreiterten Abschnitt angeordnet ist, können durch Ansteuern des Ultraschallgebers Reinigungszyklen für die Atemluftdesinfektionsvorrichtung durchgeführt werden, bei dem etwaige Schmutz-/Staubpartikel innerhalb der Atemluftdesinfektionsvorrichtung, insbesondere im verbreiterten Abschnitt abgereinigt werden können. Dies kann in gesonderten Serviceintervallen oder auch während des normalen Betriebs von Zeit zu Zeit durchgeführt werden.

Nachfolgend werden zwei Ausführungsbeispiele anhand der beiliegenden Zeichnungen detailliert beschrieben.

Darin zeigt:
- Fig. 1: eine Atemluftdesinfektionsvorrichtung in einer ersten Ausführungsform in einer teils geschnittenen Ansicht,
- Fig. 2: die in Fig. 1 dargestellte Atemluftdesinfektionsvorrichtung im Querschnitt,
- Fig. 3: in räumlicher Ansicht eine zweite Ausführungsform der Erfindung in Form einer Atemschutzmaske mit zwei Atemluftdesinfektionsvorrichtungen und
- Fig. 4: in teils geschnittener Ansicht eine Seite der in Fig. 3 dargestellten Atemschutzmaske.

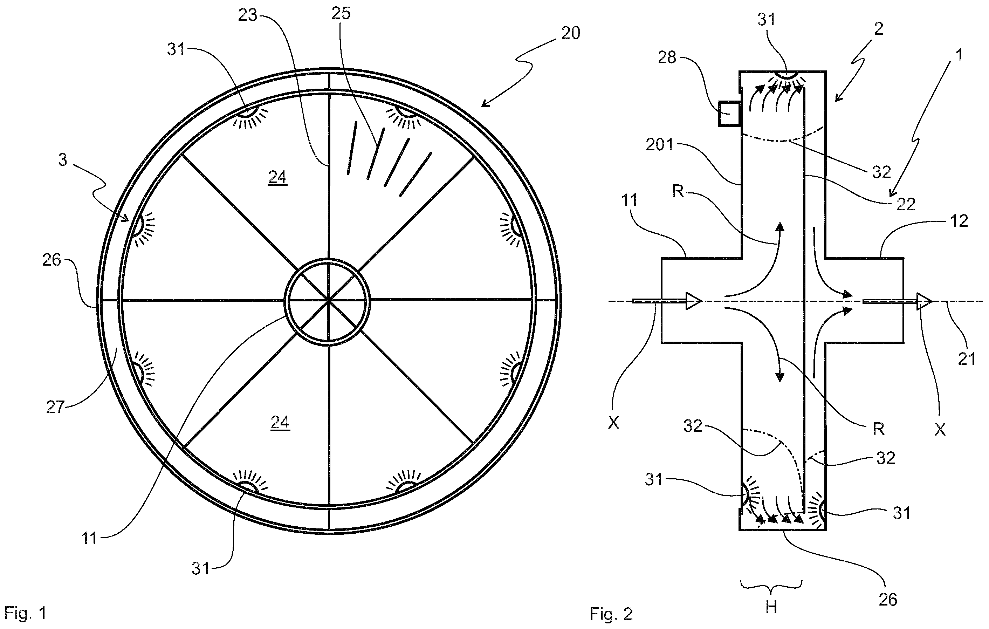

In Fig. 1 ist eine Atemluftdesinfektionsvorrichtung in einer ersten Ausführungsform in einer teils geschnittenen Ansicht dargestellt. In Fig. 2 ist diese Atemluftdesinfektionsvorrichtung im Querschnitt wiedergegeben. Dabei ist in Fig. 2 ein Atemluftkanal 1 mit einem Einlassabschnitt 11 mit geringem Durchmesser, einem verbreiterten Abschnitt 2 mit deutlich größerem Durchmesser (siehe Fig. 1) und ein Auslassabschnitt 12 wiederrum mit geringem Durchmesser dargestellt.

Der verbreiterte Abschnitt 2 des Atemluftkanals 1 ist im hier dargestellten Ausführungsbeispiel zylinderförmig oder dosenförmig ausgebildet, weist also eine relativ geringe Zylinderhöhe H und einen relativ großen Zylinderdurchmesser auf. Dabei sind zur Verdeutlichung des Strömungsweges für die durch den Atemluftkanal 1 geleitete Atemluft Pfeile mit entsprechenden Strömungsrichtungen dargestellt. Der verbreiterte Abschnitt bzw. die allseitige Aufweitung 2, hier in Zylinderform, hat eine Zylinderachse 21 die mit der ersten Fließrichtung X zusammenfällt, wobei in dem verbreiterten Abschnitt 2 in Zylinderform eine Strömungsfläche in Form einer Kreisscheibe 22 angeordnet ist. Dabei ist der verbreiterte Abschnitt 2 aus einem zylinderförmigen Gehäuse 20 gebildet. Im Einlassabschnitt 11 fließt die Atemluft mit einer ersten Fließrichtung X parallel zur Längserstreckung des Atemluftkanals 1 (entlang der Zylinderachse 21 der entsprechenden Rohrabschnitte) und teilt sich dann in zweite Fließrichtungen R, radial zur ersten Fließrichtung X wobei die Atemluftströmung sich in dem als allseitige Aufweitung in Zylinderform ausgebildeten verbreiterten Abschnitt 2 verteilt, wie mit dem dort mit R bezeichneten Strömungspfeilen angedeutet.

In der teils geschnittenen Ansicht gemäß Fig. 1 ist erkennbar, dass in dem verbreiterten Abschnitt 2 in Zylinderform Strömungsleitflächen 23 so angeordnet sind, dass der sich aufweitende Hohlraum im Inneren des Zylinders in acht gleichgroße Sektoren 24 aufgeteilt ist. Diese tortenförmigen Sektoren 24 können im äußeren Peripheriebereich zusätzlich durch zu den Strömungsleitflächen 23 ergänzenden Luftleitwänden 25 unterteilt sein.

In Fig. 1 ist in der teils geschnittenen Ansicht die Kreisscheibe 22 in der Aufsicht dargestellt. Zwischen dem äußeren kreisförmigen Rand der Kreisscheibe 22 und der umlaufenden Zylindermantelfläche 26 des Gehäuses 20 ist ein Ringraum 27 ausgebildet, über den die Atemluft um die Kreisscheibe 22 herumströmen, und wie in Fig. 2 ersichtlich, wieder zurück zur Zylinderachse 21 im Zylinder 2 und nachfolgend zum Auslassabschnitt 12 des Atemluftkanals 1 wieder in die erste Fließrichtung X umgelenkt wird.

Ferner sind in Fig. 1 auf der Zylindermantelfläche 26 jeweils innenseitig UVC-Strahlungsmittel 3 in Form von UVC-LEDs 31 dargestellt. Im hier gezeigten Ausführungsbeispiel sind für jeden Sektor 24 je eine UVC-LED 31 vorgesehen, wie dies auch in Fig. 2 oben dargestellt ist. Gegebenenfalls können im Gehäuse 20 des verbreiterten Abschnitts 2 noch weitere UVC-LEDs 31, insbesondere im Peripheriebereich, also nahe am äußeren Rand der zylinderförmigen, allseitigen Aufweitung 2 angeordnet sein, wie dies in Fig. 2 unten alternativ dargestellt ist.

Nachfolgend wird nochmals auf die Strömungswegdarstellung gemäß Fig. 2 eingegangen. Die über Einlassabschnitt 11 zufließende Atemluft in Strömungsrichtung der ersten Fließrichtung X wird durch die im verbreiterten Abschnitt 2 angeordnete Kreisscheibe 22 zwangsweise in Radialfließrichtung R in alle Radialrichtungen zur ersten Fließrichtung X umgelenkt. Entsprechend verteilt sich die Atemluft in sich fächerartig aufweitende Strömungen in den acht Sektoren 24 über den gesamten Umfang, wobei durch diese fächerartige Aufweitung über den gesamten Umfang eine Querschnittsvergrößerung im Vergleich zum Durchmesser des Einlassabschnitts 11 und bei einer entsprechenden wirksamen Höhe H des Zylinders 2 insgesamt eine deutliche Verlangsamung der Strömungsgeschwindigkeit erfolgt, wie dies durch die kürzeren Strömungspfeile am Peripherierand dargestellt ist.

Genau an dieser Stelle (am Peripherie- oder Außenrand des Zylinders 2) sind dann bevorzugt auch die UVC-Strahlungsmittel 3 in Form von einzelnen UVC-LEDs 31 angeordnet. Beispielsweise können die UVC-LEDs 31 innenseitig an der Zylindermantelfläche 26 angeordnet sein, um unmittelbar direkt auf die hierauf zuströmende Atemluft einstrahlen zu können. Gegebenenfalls können ergänzende UVC-LEDs 31 am Gehäuse 20 des Zylinders 2, insbesondere im Zylinderdeckel 201 des Gehäuses 20 wiederrum nahe am äußeren Peripheriebereich angeordnet sein. Als UVC-LED 31 können beispielsweise handelsübliche 0,8 Watt LEDs verwendet werden. Derartige UVC-LEDs 31 weisen eine Lichtintensität im UVC-Bereich auf, die zum Abtöten von Mikroorganismen, insbesondere Bakterien, Viren oder Dergleichen geeignet sind, wobei die Eindringtiefe dieser UVC-Strahlung in den Luftraum mindestens 10 mm, eher mindestens 20 mm beträgt. Entsprechend ist in Fig. 2 ein Wirkraum 32 mit strichpunktierter Linie dargestellt, in dem die von der UVC-LED 31 abgegebene UVC-Strahlung keimtötend wirkt.

Aufgrund der starken Querschnittserweiterung im Bereich des verbreiterten Abschnitts bzw. Zylinders 2, nimmt die Strömungsgeschwindigkeit entsprechend dieser Querschnittsvergrößerung ab. Aufgrund des in diesem verbreiterten Abschnitts 2 verlängerten Strömungsweges und der optimalen Einkopplung der UVC-Bestrahlung durch die UVC-Strahlungsmittel 3 im Bereich möglichst geringer Strömungsgeschwindigkeit der Atemluft, nämlich nahe am äußeren Rand des Zylinders 2, wirkt die UVC-Strahlung mit ausreichend keimtötender Wirkung über einen Bereich von 20 bis 40 mm, bei Verwendung von zwei UVC-LEDs 31 je Sektor 24 wahrscheinlich sogar über bis zu 60 mm, so dass die durch diesen Bereich langsam fließende Atemluft mit hoher Wirksamkeit aufgrund der keimtötenden Eigenschaft der UVC-Strahlung desinfiziert wird. Dabei dürften Einwirkdauern je vorbeiströmenden Luftpartikel von wenigstens einer Sekunde höchstwahrscheinlich aber mehreren Sekunden erreichbar sein.

Entsprechend wird die Keimzahl drastisch gesenkt, womit die Atemluft im Bereich des Auslassabschnittes 12 praktisch keimfrei ist.

Die Aufweitung (Vergrößerung) des Strömungsquerschnitts im Luftweg (Atemluftkanal 1) lässt sich bei dem in Fig. 1 und 2 dargestellten Ausführungsbeispiel aus dem Zylinderumfang multipliziert mit der wirksamen Zylinderhöhe im Verhältnis zum Querschnitt des Zuführungsschlauches (Einlassabschnitt 11) berechnen, beispielsweise mit
- rₛ = Radius Einlassabschnitt 11, z.B. 7,5 mm,
- r_{Z} = 5 * rₛ = Radius Zylinder 2,
- h_{Z} = 2 * * rₛ = wirksame Zylinderhöhe, also Abstand zwischen Zylinderdeckel 201 und Kreisscheibe 22,
- v₁ = 2 m/s = Strömungsgeschwindigkeit im Einlassabschnitt 11 und
- v₂ = resultierende Strömungsgeschwindigkeit nahe des Außenrandes des Zylinders

| | |
|---|---|
| ergibt sich: | π * rₛ² * v₁ = 2 * π * r_{Z *} r_{Z} * v₂ |
| | rₛ² * v₁ = 2 * 5 * rₛ * 2 * rₛ * v₂ |
| also: | v₂ = 1/20 * v₁ , |

womit die Strömungsgeschwindigkeit, die beispielsweise am Einlassabschnitt 2 m/s beträgt, nahe des Außenrandes des Zylinders auf 1/20 der Strömungsgeschwindigkeit, also 0,1 m/s reduziert wird. Und genau an dieser Stelle sind die UVC-LEDs für eine optimale Wirkung anzuordnen, wie der strichpunktiert dargestellte Wirkraum 32 in Fig. 2 verdeutlicht. Die Luft strömt hier also mit einer relativ langsamen Geschwindigkeit von 10 cm/s über einen Wirkbereich von mehreren cm, so dass die dort entlangströmende Luft für ca. 0,5 s einer intensiven UVC-Bestrahlung ausgesetzt ist.

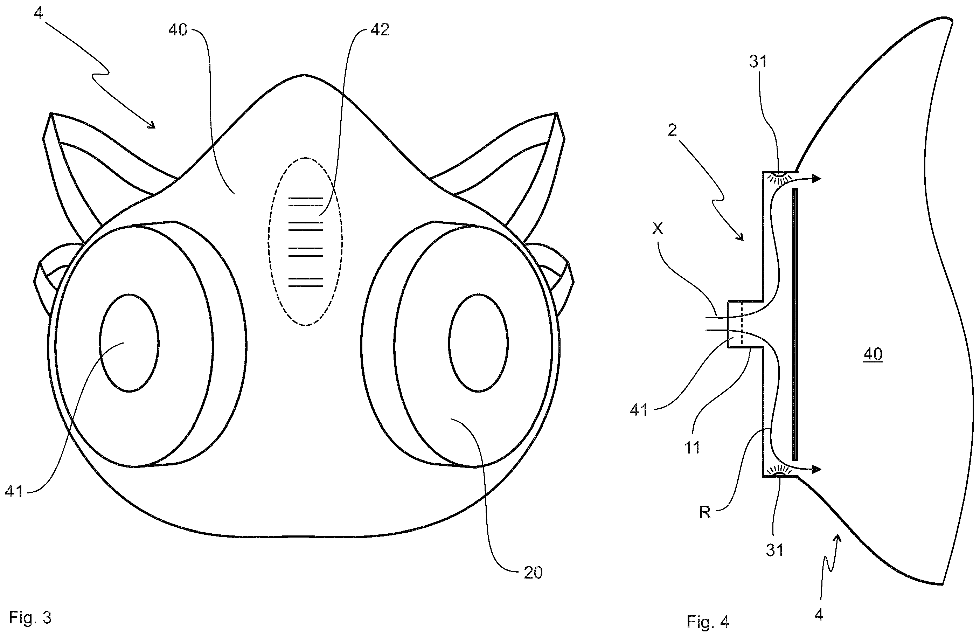

In einem zweiten Ausführungsbeispiel gemäß der Fig. 3 und 4 ist eine Atemschutzmaske 4 ausgestattet mit zwei Atemluftdesinfektionsvorrichtungen, wie beispielsweise zu den Fig. 1 und 2 beschrieben, dargestellt. In Fig. 3 ist die Atemschutzmaske 4 in räumlicher Ansicht wiedergegeben. Aus Fig. 4, in der eine teils geschnittene Teilansicht der Atemschutzmaske 4 gemäß Fig. 3 dargestellt ist, ist der Strömungsweg der Atemluft durch die jeweilige Atemluftdesinfektionsvorrichtung dargestellt. In diesem zweiten Ausführungsbeispiel sind zum ersten Ausführungsbeispiel funktionsgleiche Bauteile mit gleichen Bezugszeichen benannt.

Im Gegensatz zum ersten Ausführungsbeispiel wird die Atemluft unmittelbar nach Passieren der Kreisscheibe 22 frei in die Atemschutzmaske 4 eingeleitet. Damit wird die Atemluft vorteilhaft mit geringer Strömungsgeschwindigkeit in den Innenraum der Atemschutzmaske 4 eingeleitet, so dass kaum unangenehme Luftströmungsbewegungen an der Gesichtshaut zu merken sind und auch kaum Verwirbelungen innerhalb der Atemschutzmaske 4 auftreten. Die Atemschutzmaske 4 weist einen luftdichten Maskenkörper 40 auf, an dem die beiden Atemluftdesinfektionsvorrichtungen gemäß der Fig. 1 und 2 eingefügt sind. Am freien Ende des Einlassabschnitts 11 ist in diesem Ausführungsbeispiel jeweils ein Feinstaubfilter 41 angeordnet. Ferner ist in dem luftdichten Maskenkörper 40 ein Ausatemventil 42 untergebracht, sodass die Atemluft von der Person nach dem Ausatmen über dieses Ausatemventil 42 in Art eines Rückschlagventils direkt an die freie Umgebung abgegeben werden kann. Gegebenenfalls kann eine Sensorik in beiden Atemluftdesinfektionsvorrichtungen zur Überwachung der Verteilung des Luftstromes vorgesehen sein. Selbstverständlich kann alternativ auch die Atemluft wieder durch die Atemluftdesinfektionsvorrichtungen hindurchgeführt werden, um entsprechend bei etwaig infizierten Personen auch die an die Umgebung abgegebene Ausatemluft zu desinfizieren.

Als weitere Option kann an der Atemluftdesinfektionsvorrichtung, insbesondere am verbreiterten Abschnitt/Zylinder 2 ein Ultraschallgeber 28 vorgesehen sein, der bei Aktivierung ein Abreinigen durch die vom Ultraschallgeber 28 generierten Vibrationsbewegungen von Staub-/Schmutzpartikeln im Bereich des Atemluftkanals 1, insbesondere am verbreiterten Abschnitt/Zylinder 2 bewirkt. Diese Abreinigung kann zu vorgegebenen Zeitintervallen oder in einem Servicemodus bewirkt werden, um stets eine optimale Wirkung der UVC-Bestrahlung und gleichzeitig hygienische Innenflächen in der Atemluftdesinfektionsvorrichtung sicherzustellen.

Eine weitere Möglichkeit, um die Effizienz der UVC-Bestrahlung zu erhöhen liegt darin, dass UVC-Licht durch Modulation der Amplitude bzw. der Frequenz zu verändern und/oder das UVC-Licht mit einer oder weiteren Lichtquellen mit unterschiedlicher Frequenz zu überlagern, um ein Summenlichtsignal im UVC-Bereich zu erhalten, das eine höhere Wirkung und ggf. geringere Dämpfung in der Luft und ggf. im UVC-Lichtdurchlässigen Material des Zylinders 2 erfährt.

Dabei begünstigt die UVC-Lichtmodulation mit einer Trägerfrequenz eine weiterleitende Wirkung durch die Luft. Dabei führt diese UVC-Bestrahlung beim Auftreten spezieller kristalliner Flächen/Strukturen zu einer Demodulation und kann dort seine UVC-Wirkung wieder entfalten. Diese kristallinen Strukturen zur Demodulation können im Gehäuse 20 des Zylinders 2 angeordnet werden, um dort die Desinfektionswirkung zu begünstigen und der Dämpfung durch die Luft und ggf. im UVC-Lichtdurchlässigen Material des Zylinders 2 entgegenzuwirken.

### Bezugszeichenliste

- 1: Atemluftkanal
- 11: Einlassabschnitt
- 12: Auslassabschnitt

- 2: verbreiterter Abschnitt, allseitige Aufweitung, Zylinder
- 20: Gehäuse
- 201: Zylinderdeckel
- 21: Zylinderachse
- 22: Strömungsfläche, Kreisscheibe
- 23: Strömungsleitfläche
- 24: Sektor
- 25: Luftleitwand
- 26: Zylindermantelfläche
- 27: Ringraum
- 28: Ultraschallgeber

- 3: UVC-Strahlungsmittel
- 31: UVC-LED
- 32: Wirkraum

- 4: Atemschutzmaske
- 40: luftdichter Maskenkörper
- 41: Feinstaubfilter, Partikelfilter
- 42: Ausatemventil

- H: Höhe des Zylinders
- R: Radialfließrichtung
- X: erste Fließrichtung

## Patentansprüche

1. Atemluftdesinfektionsvorrichtung mit einem Atemluftkanal (1), über den Atemluft über einen Einlassabschnitt (11) in einer ersten Fließrichtung (X) einer Person zu- und/oder abgeführt wird, wobei ein verbreiterter Abschnitt (2) des Atemluftkanals (1) vorgesehen ist und im verbreiterten Abschnitt (2) des Atemluftkanals (1) UVC-Strahlungsmittel (3) zur Desinfektion der Atemluft angeordnet sind, wobei der verbreiterte Abschnitt (2) des Atemluftkanals (1) eine allseitige Aufweitung in alle Radialrichtungen (R) aufweist, wobei im Bereich der größten Aufweitung eine den geradlinigen Durchfluß der Atemluft hindernde Strömungsfläche (22) vorgesehen ist, die eine Umlenkung der Atemluft in alle Radialrichtungen (R) zur ersten Fließrichtung (X) nach außen in diesem verbreiterten Abschnitt (2) des Atemluftkanals (1) bewirkt, der wirksame Strömungsquerschnitt im verbreiterten Abschnitt (2) gegenüber dem Strömungsquerschnitt im Einlassabschnitt (11) um den Faktor 10 bis 500 vergrößert ist, wobei die UVC-Strahlungsmittel (3) im verbreiterten Abschnitt (2) im Bereich der größten Strömungsquerschnittsaufweitung angeordnet sind und die allseitige Aufweitung die Form eines Zylinders (2) hat, wobei die Zylinderachse (21) des Zylinders (2) mit der ersten Fließrichtung (X) und der Achse des Einlassabschnittes (11) zusammenfällt und die Strömungsfläche eine Kreisscheibe (22) ist, die mittig und orthogonal zur ersten Fließrichtung (X) im Zylinder (2) so angeordnet ist, dass ein Ringraum (27) am äußeren Rand zwischen Zylindermantelfläche (26) und Kreisscheibe (22) zur weiteren Strömungsumlenkung frei bleibt.

2. Atemluftdesinfektionsvorrichtung nach Anspruch 1, **dadurch gekennzeichnet, dass** der wirksame Strömungsquerschnitt im verbreiterten Abschnitt (2) gegenüber dem Strömungsquerschnitt im Einlassabschnitt (11) um den Faktor 20 bis 100 vergrößert ist.

3. Atemluftdesinfektionsvorrichtung nach einem der vorangehenden Ansprüche, **dadurch gekennzeichnet, dass** Strömungsleitflächen (23) im verbreiterten Abschnitt (2) des Atemluftkanals (1) vorgesehen sind, die die Strömung der Atemluft auf gleichartige Strömungswege aufteilt.

4. Atemluftdesinfektionsvorrichtung nach einem der vorangehenden Ansprüche, **dadurch gekennzeichnet, dass** der verbreiterte Abschnitt (2) des Atemluftkanals (1) aus einem UVC-Licht durchlässigen Material gebildet ist, an dem als UVC-Strahlungsmittel (3) UVC-LEDs (31) angeordnet sind.

5. Atemluftdesinfektionsvorrichtung nach einem der vorangehenden Ansprüche, **dadurch gekennzeichnet, dass** ein Flüssigkeitsabscheider und/oder ein Partikelfilter (41) im Atemluftkanal (1) in Strömungsrichtung der Atemluft vor dem verbreiterten Abschnitt (2) angeordnet ist.

6. Atemluftdesinfektionsvorrichtung nach einem der vorangehenden Ansprüche, **dadurch gekennzeichnet, dass** ein Ultraschallgeber (28) am verbreiterten Abschnitt (2) angeordnet ist.

7. Atemschutzmaske (4) mit wenigstens einer Atemluftdesinfektionsvorrichtung nach einem der vorangehenden Ansprüche.

8. Atemluftdesinfektionsverfahren, mit dem Atemluft über einen Atemluftkanal (1) in einer ersten Fließrichtung (X) einer Person zu- und/oder abgeführt wird, wobei die im Atemluftkanal (1) strömende Atemluft einer UVC-Strahlung ausgesetzt wird, mit den Schritten:
- Umlenken der Atemluft in alle Radialrichtungen (R) zur ersten Fließrichtung (X) nach außen in einem verbreiterten Abschnitt (2) des Atemluftkanals (1), womit der Strömungsweg und der Strömungsquerschnitt in diesem verbreiterten Abschnitt (2) des Atemluftkanals (1) vergrößert wird, und
- Bestrahlen der Atemluft in diesem verbreiterten Abschnitt (2) des Atemluftkanals (1) mit UVC-Strahlung, wobei die Atemluft am radial-äußeren Rand im Bereich der größten Aufweitung des Strömungsquerschnittes der UVC-Strahlung ausgesetzt wird.

9. Atemluftdesinfektionsverfahren nach Anspruch 8, **dadurch gekennzeichnet, dass** die Atemluft am radial-äußeren Rand in diesem verbreiterten Abschnitt (2) des Atemluftkanals (1) wieder in die erste Fließrichtung (X) rückumgelenkt wird.

10. Atemluftdesinfektionsverfahren nach Anspruch 8 oder 9, **dadurch gekennzeichnet, dass** die UVC-Strahlung in der Strahlungsfrequenz moduliert wird.

11. Atemluftdesinfektionsverfahren nach Anspruch 8, 9 oder 10, **dadurch gekennzeichnet, dass** die UVC-Strahlung aufsummiert wird.

## Claims

1. Respiratory air disinfection device having a respiratory air channel (1), through which respiratory air is conducted to and/or from a person via an inlet portion (11) in a first direction of flow (X), wherein a widened section (2) of the breathing air channel (1) is provided and UVC radiation means (3) for disinfecting the breathing air are arranged in the widened section (2) of the breathing air channel (1), wherein the widened section (2) of the breathing air channel (1) widens omnidirectionally in all radial directions (R) , wherein in the area of the greatest widening a flow surface (22) is provided which prevents the breathing air from flowing through in a straight line and which causes deflection of the breathing air in all radial directions (R) to the first flow direction (X) outwards in this widened section (2) of the breathing air duct (1), the effective flow cross-section in the widened section (2), compared to the flow cross-section in the inlet section (11), is enlarged by a factor of 10 to 500, wherein the UVC radiation means (3) are arranged in the widened section (2) in the area of the largest flow cross-section expansion and the omnidirectional expansion has the shape of a cylinder (2), wherein the cylinder axis (21) of the cylinder (2) coincides with the first flow direction (X) and the axis of the inlet section (11), and the flow surface is a circular disc (22) which is arranged centrally and orthogonally to the first flow direction (X) in cylinder (2) in such a way that an annular space (27) remains free at the outer edge between cylinder housing surface (26) and circular disc (22) for further flow deflection.

2. Respiratory air disinfection device according to claim 1, **characterized in that** the effective flow cross-section in the widened section (2), compared to the flow cross-section in the inlet section (11), is increased by a factor of 20 to 100.

3. Respiratory air disinfection device according to any one of the preceding claims, **characterized in that** baffle surfaces (23) are provided in the widened section (2) of the respiratory air channel (1), which divide the flow of the respiratory air to equal flow paths.

4. Respiratory air disinfection device according to any one of the preceding claims, **characterized in that** the widened section (2) of the respiratory air channel (1) is formed from a UVC light-transmitting material on which UVC LEDs (31) are arranged as UVC radiating means (3).

5. Respiratory air disinfection device according to any one of the preceding claims, **characterized in that** a liquid separator and/or a particle filter (41) is arranged in the respiratory air channel (1) in front of the widened section (2) in the direction of flow of the respiratory air.

6. Respiratory air disinfection device according to any one of the preceding claims, **characterized in that** an ultrasonic transducer (28) is arranged on the widened section (2).

7. Respiratory protection mask (4) with at least one respiratory air disinfection device according to one of the preceding claims.

8. Respiratory air disinfection method by which respiratory air is supplied and/or discharged via a respiratory air channel (1) in a first direction of flow (X) to a person, wherein the respiratory air flowing in the respiratory air channel (1) is exposed to UVC radiation, with the steps:
- redirecting the respiratory air in all radial directions (R) to the first flow direction (X) outwards in a wider section (2) of the respiratory air channel (1), whereby the flow path and the flow cross-section in this widened section (2) of the respiratory air channel (1) increase and
- irradiating the respiratory air in this widened section (2) of the respiratory air channel (1) with UVC radiation, wherein the respiratory air is exposed to UVC radiation at the radial-outer edge in the area of the largest expansion of the flow cross-section.

9. Respiratory air disinfection method according to claim 8, **characterized in that** the respiratory air at the radial-outer edge in this widened section (2) of the respiratory air channel (1) is redirected back to the first flow direction (X).

10. Respiratory air disinfection method according to claim 8 or 9, **characterized in that** the UVC radiation is modulated in the radiation frequency.

11. Respiratory air disinfection method according to claim 8, 9 or 10, **characterized in that** the UVC radiation is summed.

## Revendications

1. Dispositif de désinfection de l'air respiré comprenant un canal d'air respiré (1), par lequel l'air respiré est amené à une personne et/ou écarté de celle-ci via une section d'entrée (11) dans une première direction d'écoulement (X), une section élargie (2) du canal d'air respiré (1) étant prévue, et des moyens de rayonnement UVC (3) étant disposés dans la section élargie (2) du canal d'air respiré (1) pour la désinfection de l'air respiré, la section élargie (2) du canal d'air respiré (1) présentant un élargissement sur tous les côtés dans toutes les directions radiales (R), une surface d'écoulement (22) gênant l'écoulement rectiligne de l'air respiré étant prévue dans cette section élargie (2) du canal d'air respiré (1), dans la zone du plus grand élargissement, laquelle surface d'écoulement provoque une déviation de l'air respiré dans toutes les directions radiales (R) vers l'extérieur par rapport à la première direction d'écoulement (X), la section transversale d'écoulement effective dans la section élargie (2) étant augmentée d'un facteur 10 à 500 par rapport à la section transversale d'écoulement dans la section d'entrée (11), les moyens de rayonnement UVC (3) étant disposés dans la section élargie (2) dans la zone du plus grand élargissement de la section transversale d'écoulement, et l'élargissement sur tous les côtés ayant la forme d'un cylindre (2), l'axe de cylindre (21) du cylindre (2) coïncidant avec la première direction d'écoulement (X) et avec l'axe de la section d'entrée (11), et la surface d'écoulement étant un disque circulaire (22) disposé de manière centrale et orthogonale à la première direction d'écoulement (X) dans le cylindre (2), de sorte qu'un espace annulaire (27) demeure libre au bord extérieur entre la surface d'enveloppe du cylindre (26) et le disque circulaire (22) pour une déviation supplémentaire de l'écoulement.

2. Dispositif de désinfection de l'air respiré selon la revendication 1, **caractérisé en ce que** la section transversale d'écoulement effective dans la section élargie (2) est augmentée d'un facteur 20 à 100 par rapport à la section transversale d'écoulement dans la section d'entrée (11).

3. Dispositif de désinfection de l'air respiré selon l'une des revendications précédentes, **caractérisé en ce que** des surfaces de guidage d'écoulement (23) sont prévues dans la section élargie (2) du canal d'air respiré (1), lesquelles répartissent le flux d'air respiré sur des voies de flux similaires.

4. Dispositif de désinfection de l'air respiré selon l'une des revendications précédentes, **caractérisé en ce que** la section élargie (2) du canal d'air respiré (1) est formée d'un matériau perméable à la lumière UVC, sur lequel des DEL UVC (31) sont disposées en tant que moyens de rayonnement UVC (3).

5. Dispositif de désinfection de l'air respiré selon l'une des revendications précédentes, **caractérisé en ce qu'**un séparateur de liquide et/ou un filtre à particules (41) est disposé dans le canal d'air respiré (1), en amont, dans la direction d'écoulement de l'air respiré, de la section élargie (2).

6. Dispositif de désinfection de l'air respiré selon l'une des revendications précédentes, **caractérisé en ce qu'**un émetteur à ultrasons (28) est disposé au niveau de la section élargie (2).

7. Masque de protection respiratoire (4) comprenant au moins un dispositif de désinfection de l'air respiré selon l'une des revendications précédentes.

8. Procédé de désinfection de l'air respiré, dans lequel l'air respiré est amené à une personne et/ou écarté de celle-ci via un canal d'air respiré (1) dans une première direction d'écoulement (X), l'air respiré circulant dans le canal d'air respiré (1) étant exposé à un rayonnement UVC, comprenant les étapes suivantes consistsant à :
- dévier l'air respiré dans toutes les directions radiales (R) vers l'extérieur par rapport à la première direction d'écoulement (X) dans une section élargie (2) du canal d'air respiré (1), ce qui augmente le trajet d'écoulement et la section transversale d'écoulement dans cette section élargie (2) du canal d'air respiré (1), et
- irradier l'air respiré dans cette section élargie (2) du canal d'air respiré (1) avec un rayonnement UVC, l'air respiré étant exposé au rayonnement UVC au niveau du bord radialement extérieur dans la zone du plus grand élargissement de la section transversale d'écoulement.

9. Procédé de désinfection de l'air respiré selon la revendication 8, **caractérisé en ce que** l'air respiré est à nouveau dévié dans la première direction d'écoulement (X) au niveau du bord radialement extérieur dans cette section élargie (2) du canal d'air respiré (1).

10. Procédé de désinfection de l'air respiré selon la revendication 8 ou 9, **caractérisé en ce que** le rayonnement UVC est modulé en fréquence de rayonnement.

11. Procédé de désinfection de l'air respiré selon la revendication 8, 9 ou 10, **caractérisé en ce que** le rayonnement UVC est cumulé.
